Europäisches Patentamt

European Patent Office

Office européen des brevets

(19)

(11) Veröffentlichungsnummer: **0 055 868**
**A2**

(12) # EUROPÄISCHE PATENTANMELDUNG

(21) Anmeldenummer: **81110852.1**

(22) Anmeldetag: **30.12.81**

(51) Int. Cl.[3]: **G 01 N 33/54**
**G 01 N 33/58**

(30) Priorität: **02.01.81 DE 3100061**

(43) Veröffentlichungstag der Anmeldung:
**14.07.82 Patentblatt 82/28**

(84) Benannte Vertragsstaaten:
**AT BE CH DE FR GB IT LI LU NL SE**

(71) Anmelder: **Thoma, Hans A., Dr.**
**Giselastrasse 3**
**D-8000 München 40(DE)**

(72) Erfinder: **Thoma, Hans A., Dr.**
**Giselastrasse 3**
**D-8000 München 40(DE)**

(74) Vertreter: **Hansen, Bernd, Dr.rer.nat. et al,**
**Hoffmann . Eitle & Partner Patentanwälte**
**Arabellastrasse 4**
**D-8000 München 81(DE)**

(54) **Verfahren zur Bestimmung von Liganden oder Rezeptoren mittels Kompetitionsreaktion, sowie Test-Kit zur Durchführung desselben und Verwendung.**

(57) Es wird ein immunologisches Verfahren zur Bestimmung von Liganden oder Rezeptoren mittels Kompetitionsreaktion und unter Verwendung eines spezifisch bindenden Rezeptor A und eines Konjugates beschrieben, wobei das Verfahren folgende Schritte umfasst:

a) Als Konjugat wird der Testprobe ein Marker-Rezeptor A-Konjugat zugegeben und

b) zur Bindung der Marker-Komponente von nicht-gebundenem Marker-Rezeptor A-Konjugat wird der Probe ein spezifisch bindender Rezeptor zugegeben.

c) zur Sättigung freier Bindungsstellen am Rezeptor B werden Marker-Moleküle zugegeben und

d) die Menge an nicht-gebundenen Marker-Molekülen wird als Signalmolekül bestimmt.

Ausserdem wird ein Test-Kit zur Durchführung des erfindungsgemässen Verfahrens sowie dessen Verwendung beschrieben. Das Verfahren gemäss der Erfindung erlaubt eine besonders genaue und schnelle Bestimmung von Liganden und Rezeptormolekülen auf homogene Weise.

- 1 -

Verfahren zur Bestimmung von Liganden oder Rezeptoren mittels Kompetitionsreaktion, sowie Test-Kit zur Durchführung desselben und Verwendung

Die Erfindung betrifft ein Verfahren zur Bestimmung von Liganden oder Rezeptoren mittels Kompetitionsreaktion unter Verwendung von spezifisch bindendem Rezeptor A und einem Konjugat, sowie ein Test-Kit zur Durchführung desselben.

In den vergangenen Jahren sind zahlreiche Methoden entwickelt worden, um biologisch aktive Substanzen zu bestimmen. Insbesondere besteht in der klinischen Chemie ein Bedürfnis, diagnostisch wichtige Substanzen, wie Wirkstoffe von Arzneimitteln, Drogen oder Narkotika, Hormone, Polypeptide, Stoffwechselprodukte, Toxine, etc., in Körperflüssigkeiten, wie Serum oder Harn, bestimmen zu können. Dabei ist es wichtig, dass diese Bestimmungsmethoden schnell und einfach vom Laborpersonal durchgeführt werden können. Da die genannten Substanzen häufig nur in geringen Konzentrationen vorliegen, ist es wesentlich, dass diese Nachweismethoden äusserst empfindlich sind.

Unter anderem wurde eine Reihe von Methoden entwickelt, die auf der Fähigkeit eines Rezeptors, im allgemeinen eines Antikörpers, beruhen, mit einem Liganden einen Komplex hoher molekularer Spezifität zu bilden. Während die Spezifität dieser Methoden im wesentlichen auf der Schlüssel-Loch-Komplementarität beruht, ist die Empfindlichkeit von der Komplexbildungskonstante (thermodynamische Gleichgewichtskonstante) von Rezeptor und Ligand abhängig. Bei Verwendung eines markierten bzw. modifizierten Liganden und Durchführung einer Kompetitionsreaktion von markiertem und unmarkiertem Liganden um die freien Bindungsstellen, kann - sofern die Ligandenkonzentration in der Probe die einzige Variable des Testsystems darstellt - aus dem Verhältnis zwischen freiem und Antikörper-gebundendem markierten Liganden auf die unbekannte Konzentration des Liganden in der Probe geschlossen werden. Zur Auswertung ist es dann erforderlich, dass entweder

(a)     freier und Antikörper-gebundener, markierter Ligand durch geeignete Verfahren getrennt werden, z.B. heterogene Methoden, wie RIA oder ELISA, oder

(b)     freier und Rezeptor-gebundener, markierter Ligand unterschiedliche Signale ergeben.

Als Markierung für den Liganden sind eine Reihe von Stoffgruppen mit speziellen physikochemischen oder biochemischen Eigenschaften beschrieben worden. Insbesondere wurden die Liganden mit Markierungssubstanzen, wie Radioisotope, Enzyme, Phagen, fluoreszierende Substanzen und freie Radikale, umgesetzt.

Diese Methoden nach dem Stand der Technik weisen jedoch zahlreiche Nachteile auf. So sind z.B. bei den radioimmunologischen Methoden die Gefahren der Handhabung radioaktiv-markierter Substanzen unter Berücksichtigung der entsprechenden behördlichen Auflagen hinderlich. Der Einsatz fluoreszierender Komponenten ist aufgrund sich ergebender Verstärkungsprobleme begrenzt. Andere Verfahren sind in ihrer Durchführung entweder zu aufwendig, nicht routinemässig durchführbar oder weisen Probleme mangelnder Sensibilität oder zu geringer Spezifität auf.

In jüngerer Zeit ist auch der Einsatz von reversibel bindendem Enzymmodulator, der sich in nicht kovalenter Weise mit einem Enzym umsetzt, in der DE-OS 27 54 086 beschrieben worden. Durch analytische Bestimmung des Ausmasses der hemmenden oder stimulierenden Wirkung des Modulators auf die Enzymaktivität können dessen Konzentration bestimmt und Rückschlüsse auf unbekannte Konzentration des Probenliganden gezogen werden.

Dieses System überwindet jedoch nur die Nachteile der primären Bindungsreaktion, an welcher der Ligand und der markierte Ligand beteiligt sind, und überträgt sie auf das Detektorsystem, wobei als Signalmolekül ein markierter Ligand dient.

Der vorliegenden Erfindung liegt die Aufgabe zugrunde, eine neue Bestimmungsmethode für Liganden und Rezeptoren zur Verfügung zu stellen, mit welcher die Nachteile der Verfahren nach dem Stand der Technik überwunden werden. Es wird ein Verfahren zur Verfügung gestellt, wobei

- 4 -

die analytische Bestimmung an einem nicht-markierten bzw. nicht-modifizierten Molekül als Signalmolekül erfolgt. Insbesondere wird ein Verfahren geschaffen, womit auch hochmolekulare Substanzen durch eine homogene, nicht-radioaktive Methode bestimmt werden können. Durch Verwendung eines weiteren spezifischen Rezeptors, welcher gegen die Markerkomponente des Marker-Rezeptor-Konjugates gerichtet ist, sollen die bestehenden Analysemethoden zum Nachweis von Liganden, wie Haptenen, Antigenen, etc., wie auch Rezeptoren, bereichert und verbessert werden. Mit Hilfe des neuen Verfahrens soll eine hohe Ansprechbarkeit im p-Mol-Bereich gewährleistet sowie die Möglichkeit gegeben werden, die Bestimmungen mit hoher Genauigkeit möglichst störungsfrei auf homogene Weise durchzuführen.

Die erfindungsgemässe Aufgabe wird dadurch gelöst, dass ein Verfahren zur Bestimmung von Liganden oder Rezeptoren zur Verfügung gestellt wird, welches dadurch gekennzeichnet ist, dass

(a) als Konjugat ein Marker-Rezeptor A-Konjugat und

(b) zur Bindung der Markerkomponente von nicht reagiertem Marker-Rezeptor A-Konjugat, ein spezifisch bindender Rezeptor B zugegeben wird,

(c) gegebenenfalls freie Bindungsstellen am Rezeptor B durch Zugabe von Markermolekülen abgesättigt werden, und

(d) die Menge von nicht-gebundenem Marker als Signalmolekül analytisch bestimmt wird.

Darüber hinaus wird gemäss der Erfindung die gleichzeitige Verwendung von spezifisch bindenden Rezeptoren A und B vorgeschlagen, wobei der Rezeptor A die Ligandkomponente, der Rezeptor B die Markerkomponente im Marker-Rezeptor A-Konjugat zu binden vermag, zur Bestimmung von Liganden, insbesondere diagnostisch wichtigen Substanzen.

Durch die Schaffung von Test-Kits, welche neben den Rezeptoren A und B, einem Marker-Rezeptor A-Konjugat, Markersubstanz sowie Substanzen des Nachweissystems zur Bestimmung des Markers enthalten können, wird eine problemlose und präzise Bestimmungsmethode, welche sich für die routinemässige Durchführung in klinischen Labors eignet, zur Verfügung gestellt.

Das erfindungsgemässe Verfahren ist besonders vorteilhaft, da - im Gegensatz zu den bekannten Verfahren - keine Limitierung in chemischer Hinsicht gegeben ist, d.h. bei der Bildung des Antikörpers jeweils die gleiche Kopplungschemie zugrundeliegt. Die Kopplung von Ligand/Makromolekül zum Antigen erfolgt vorzugsweise nach der gleichen Kopplungschemie wie die Kopplung Marker-Rezeptor A.

Ausserdem ist es gemäss der Erfindung vorteilhaft, dass keine Limitierung hinsichtlich des Signalmoleküls gegeben ist. Es kann jedes Signalmolekül verwendet werden, da gemäss der Erfindung nicht die direkte Bestimmung des Konjugates sondern des freien Signalmoleküls erfolgt.

Ein besonderer Vorteil des erfindungsgemässen Verfahrens ist die homogene Bestimmung. In dieser Hinsicht ist das erfindungsgemässe Verfahren ebenfalls vorteilhaft gegenüber bekannten Verfahren, welche in irgendeiner Weise direkt das Konjugat messen und auf bestimmte Signalmoleküle limitiert sind, die nach der Konjugation noch als Signal aktiv sind.

Mit der Bezeichnung "Ligand" sollen hier grundsätzlich all diejenigen Liganden erfasst sein, die in der DE-AS 22 23 385 in den Spalten 22 bis 46 beschrieben worden sind, worauf ausdrücklich für die Zwecke der vorliegenden Erfindung Bezug genommen wird. Der Ligand kann in seinem Molekulargewicht stark variieren und zwar von etwa 100 bis 1.000.000. Dieser Molekulargewichtsbereich wird durch das erfindungsgemässe Verfahren erfasst, vorausgesetzt, es steht für die Primärreaktion ein Rezeptor zur Verfügung, der den Liganden spezifisch bindet.

Lediglich zur Erläuterung sei hier ausgeführt, dass es sich hierbei unter anderem um Antigene, um Haptene oder auch um natürlich vorkommende Rezeptoren handeln kann. Unter diese Gruppe fallen eine Fülle biologisch aktiver Substanzen, wie Hormone, z.B. die Schilddrüsenhormone $T_3$ und $T_4$, zahlreiche Steroide, wie Östrogene, Cortison, Corticosteron oder Östradiol, Peptide, Polypeptide, wie Insulin, Lipoproteine, Glycoproteine, Sterine, Nucleinsäuren, Mono- und Polysaccharide, Alkaloide, Vitamine, Enzyme, Coenzyme, aber auch Wirkstoffe von Arzneimitteln, häufig eingenommene Drogen, wie Antibiotika, sowie Toxine, Pestizide, celluläre oder extracelluläre Gewebekomponenten und aus Menschen oder Tieren isolierte Antikörper.

Als Komponenten, die mit Hilfe des erfindungsgemässen Verfahrens bestimmt werden können, sind insbesondere zu nennen: $T_3$, $T_4$, Analgetika, wie Morphin, Amphetamin, Gentamycin, Barbiturate, Pharmaka, wie Digoxin, Digitoxin, Phenobarbital, Diphenylhydantoin, Vitamine, Nahrungsmittelschadstoffe, Herbizide, Insektizide, Tumor-Antigene, Rheumatoid-Faktor, Myoglobin,, Ferritin, etc.. Ausserdem ist das erfindungsgemässe Verfahren anwendbar zur Bestimmung von TSH, HCG, IgG, Hepatitis-Antigen, etc..

Bei der Bezeichnung "Rezeptor" handelt es sich um eine spzifisch bindende Substanz, insbesondere Antikörper sowie spezifisch bindende Proteine, wie z.B. Transportproteine. Die Gewinnung der Antikörper für die Primär- und Sekundärreaktion erfolgt beispielsweise durch Immunisierung von Tieren mit entsprechend hochgereinigtem Antigen, Carrier-gebundenem Hapten, etc..

Die Antikörper sind häufig Gammaglobuline, die mit hoher Spezifität den entsprechenden Liganden zu binden vermögen. Unter der Bezeichnung Rezeptor swllen hier neben normalen Antikörpern auch sogenannte Anti-Inhibitor-Antikörper, gegebenenfalls in Kombination mit Anti-IgG-Antikörpern und/oder auch Fab-Fragmente verstanden werden.

Der Begriff "Marker-Rezeptor A-Konjugat" bedeutet ein Konjugat aus einem Rezeptor A, wie es vorstehend beschrieben wird, und einer Markerkomponente.

Das Marker-Rezeptor A-Konjugat wird im allgemeinen nach Methoden erzeugt, wie sie für die Bindung anderer Markersubstanzen, wie z.B. Enzym-, Coenzym-Marker, Verwendung finden. Die Kopplungsreaktion hängt im wesentlichen von den am Rezeptor A bzw. am Markermolekül zur Verfügung stehenden bzw. einführbaren reaktiven bzw. aktivierbaren Gruppen ab. Im allgemeinen werden die bekannten Methoden der Peptidchemie etc. angewandt. Mit Vorteil kann man z.B. bei kleinen Haptenen analoge Reaktionen zur Synthese des Antigens, z.B. Kopplung an Albumin, Thyroglobulin, verwenden. Vorteilhaft sind auch Kopplungsverfahren mit bifunktionellen Reagentien, wie z.B. Carbodiimiden (z.B. 1-Cyclohexyl-3-(morpholinethyl)-carbodiimid), Glutaraldehyd, m-Maleinimidobenzyl-N-hydroxysuccinimidester, etc., sowie spezielle Verfahren, wie die reduktive Aminierung, gemischte Anhydridmethoden, Thioetherverfahren, etc..

Wesentlich ist bei der Bildung des Marker-Rezeptor A-Konjugates, dass auch nach Durchführung der Kopplungsreaktion die Markerkomponente im Konjugat zur Bindung mit dem spezifisch bindenden Rezeptor B befähigt bleibt, und nicht etwa durch den Rezeptor A-Anteil im Konjugat eine diesbezüglich sterische Hinderung erfährt. Aus der Immunologie ist bereits bekannt, dass der Antikörper nur für den im Antigen exponierten Teil spezifisch ist und verschiedene Konjugate, die nach der gleichen Kopplungsreaktion hergestellt werden, bindet.

Als Markerkomponente im Marker-Rezeptor A-Konjugat kommen solche Substanzen in Frage, die allein oder zusammen mit einem Carrier-Molekül die Bildung eines Antikörpers induzieren können oder für welche ein spezifisch bindender Rezeptor bekannt ist.

Als Markersubstanzen sind insbesondere zu nennen: alle Moleküle, die als Signalmolekül besonders geeignet sind, Enzymmodulatoren, wie Enzymaktivatoren oder -inhibitoren, Enzyme, Enzymsubstrate, Coenzyme, wie NADPH, ATP, Peptide, Antigene, Biotin, Polypeptide, sowie Substanzen, welche durch spezifische Rezeptoren gebunden werden, z.B. durch Transportproteine. Dabei muss das Markermolekül nicht Signalmolekül sein; wesentlich ist nur, dass sowohl das Markermolekül, als auch das Signalmolekül spezifisch binden. Rezeptor B könnte einen Antikörper gegen Testosteron darstellen, dann wäre Testosteron der Marker, während Signalmolekül, z.B. ein fluoreszierendes Derivat von Testosteron oder eine Reaktionsfolge, in der Testosteron quantitativ involviert ist, sein kann.

Obwohl vorzugsweise ein Ligand-Rezeptor-Konjugat verwendet wird, kann für spezielle immunologische Techniken, wie z.B. die Solid-Phase-Methode, auch ein Konjugat-Ligand —Marker verwendet werden.

Da sich nach einer besonderen Ausführungsform gemäss der Erfindung der Marker durch seine chemische Kopplung an den Rezeptor A immer in relativer Nähe des Rezeptors A befindet, wird nach Komplexierung mit dem korrespondierenden Liganden an Rezeptor A die Bindung mit Rezeptor B sterisch gehindert.

Somit ist eine homogene Arbeitsweise, d.h. ohne Trennschritt, gegeben, da die Komplexierung des Konjugates mit dem Liganden die Bindungsfähigkeit der Markerkomponente am zweiten Antikörper verändert, was als Signalunterschied erfasst werden kann. Es ist ein besonderer Vorteil dieser Methode, dass sich die sterische Hinderung ohne weitere Zusätze erreichen lässt.

Bei kleineren Molekülen lässt sich die sterische Hinderung erreichen, indem durch zusätzliche Vergrösserung des kleinen Liganden an einen makromolekularen Träger die gleichzeitige Bindung von mehreren Rezeptor A-Konjugaten bzw. Rezeptor A-Molekülen ermöglicht wird und somit die sterische Hinderung für Rezeptor B mit dem Markeranteil des Konjugates gegeben wird.

Gemäss der Erfindung braucht das Konjugat keine Signalaktivität zu besitzen; es ist besonders vorteilhaft, wenn das Konjugat keine Signalaktivität aufweist.

Unter dem "Signalmolekül" wird gemäss der Erfindung eine Substanz verstanden, wie sie vorstehend für die Verwendung als Marker-Substanz beschrieben wurde. Wichtig ist dabei, dass für das Signalmolekül ein spezifisch bindender Rezeptor bekannt ist bzw. ein solcher immunologisch induziert werden kann. Da die Signalsubstanz durch ein beliebiges analytisches Verfahren unmittelbar bestimmt wird, besteht ein wesentlicher Vorteil darin, dass diese Signalsubstanz ein nichtmarkiertes bzw. nicht-modifiziertes Molekül darstellt.

Als Bestimmungsmethode eignet sich jede Nachweismethode, die eine präzise und einfache Bestimmung der Menge an freiem Signalmolekül erlaubt, so unter anderem die Methoden des Recycling, der Biolumineszenz, der Fluoreszenzmessung, der Enzymaktivitätsmessung, etc.. In diesem Zusammenhang wird besonders auf die ATP-Bestimmung mittels Biolumineszen sowie auf die Bestimmung von freiem NADPH mittels Recycling-Techniken hingewiesen.

Lediglich erläuternd sei hier noch angeführt, dass sich als analytische Nachweismethode prinzipiell jede Bestimmungsmethode eignet, die rasch und präzise durchzuführen ist und mit welcher auch noch äusserst geringe Konzentrationen erfasst werden können.

Unter dem Rezeptor B soll gemäss der Erfindung eine den Marker oder die Markerkomponente im Marker-Rezeptor A-Konjugat spezifisch bindende Substanz verstanden werden. Dieser Rezeptor B wird vorzugsweise ein Antikörper sein, kann jedoch davon abgesehen jede beliebige, den Marker bindende Substanz darstellen, so unter anderem ein Transportprotein, einen Chelatbildner. Dieser Rezeptor B kann eine niedermolekulare oder vorzugsweise hochmolekulare Substanz darstellen.

Sofern der Rezeptor B ein Antikörper ist, erfolgt dessen immunologische Induzierung sowie Isolierung nach den üblichen dem Fachmann bekannten Methoden.

Mittels des Rezeptors B erfolgt somit eine zweite Kompetitionsreaktion um die Markerkomponente im Konjugat bzw. nicht-modifiziertes Markermolekül.

Das erfindungsgemässe Verfahren wird nachstehend anhand von einigen nicht optimierten Versuchsbeispielen zur Herstellung des Konjugates und zur Ermittlung von entsprechenden Eichkurven erläutert. Hierbei können die Versuchsbedingungen zur Herstellung des Konjugates, zur Induzierung der Rezeptoren A und B, etc., in der dem Fachmann bekannten Art und Weise je nach Wahl des speziellen Nachweis- bzw. Detektorsystems gewählt werden.

Im allgemeinen ergeben sich die folgenden Reaktionszeiten für die einzelnen Schritte:

(a)     bei Haptenen: Reaktionszeit Rezeptor/Ligand
        1 Minute bis 30 Stunden;
        Reaktionszeit für die Sekundärreaktion 0 Minuten bis 30 Stunden;

(b)     für grössere Liganden, z.B. Antigene: Reaktionszeit Rezeptor/Ligand 3 Minuten bis 48 Stunden;
        Reaktionszeit für die Sekundärreaktion 0 Minuten bis 48 Stunden.

Das Verfahren zur Bestimmung des Liganden, z.B. eines Haptens oder Antigens, in einer biologischen Probe kann sich durch folgende Schritte auszeichnen:

I. Bestimmung eines Liganden mit einem Mol.-Gewicht $> 100.000$:

(a) Der Probe wird eine bestimmte Menge (Überschuss) eines Marker-Rezeptor A-Konjugats bzw. ein Gemisch desselben mit Rezeptor A zugesetzt, wobei diese gegenüber dem Liganden in der Probe spezifisch sind, um einen Ligand-Rezeptor A-Komplex zu bilden.

(b) Zu dem Gemisch (a) erfolgt Zugabe einer bekannten Menge an Rezeptor B, wobei diese Menge im Überschuss gegenüber dem nicht-ligandenkomplexierenden Anteil an Marker-Rezeptor A-Konjugat vorliegt.

(c) Zu dem Gemisch (b) wird eine bekannte Menge an Signalmolekül zugegeben, wodurch freie Bindungsstellen am Rezeptor B abgesättigt werden, und die Menge der nicht-gebundenen Signalmoleküle wird durch geeignete analytische Methoden bestimmt.

II. Bestimmung eines Haptens mit einem Mol.-Gewicht $< 100.000$:

(a) Der Probe wird eine bestimmte Menge (Überschuss) eines Marker-Rezeptor A-Konjugats bzw. ein Gemisch desselben mit Rezeptor A - wobei diese gegenüber dem Liganden in der Probe spezifisch sind - und
eine bestimmte Menge eines homologen Polyepitops des Liganden zugesetzt, um einen Ligand-Rezeptor A-Komplex zu
bilden.

(b)     Zu dem Gemisch (a) erfolgt Zugabe einer bekannten Menge an Rezeptor B, wobei diese Menge im
Überschuss gegenüber dem nicht-ligandenkomplexierenden
Anteil an Marker-Rezeptor A-Konjugat vorliegt.

(c)     Zu dem Gemisch (b) wird eine bekannte Menge
an Signalmolekül zugegeben, wodurch freie Bindungsstellen am Rezeptor B abgesättigt werden, und die Menge
der nicht-gebundenen Signalmoleküle durch geeignete
analytische Methoden bestimmt.

III.    Eine weitere bevorzugte Variante des erfindungsgemässen Verfahrens ist wie folgt gekennzeichent:

(a)     Der Probe wird eine bestimmte Menge (Überschuss) eines Marker-Rezeptor A-Konjugats bzw. ein
Gemisch desselben mit Rezeptor A zugesetzt, wobei der
Rezeptor A bzw. der Rezeptor A im Konjugatmolekül
durch Zugabe von Anti-Rezeptor A-Antikörpern bzw.
Fab-Fragmenten einer anderen Tierspezies vergrössert
wurde.

(b)     Zu dem Gemisch (a) erfolgt Zugabe einer bekannten Menge an Rezeptor B, der ebenfalls durch Zugabe von Anti-Rezeptor B-Antikörpern bzw. Anti-Rezeptor B-Fab-Fragmenten sterisch vergrössert wird.

(c)     Zu dem Gemisch (b) wird eine bekannte Menge an Signalmolekül zugegeben, wodurch freie Bindungsstellen am Rezeptor B abgesättigt werden, und die Menge der nicht-gebundenen Signalmoleküle durch geeignete analytische Methoden bestimmt.

IV.     Bestimmung eines Rezeptors (Rezeptor A)

a) Zu der Probe wird eine bekannte Menge eines Marker-Rezeptor A-Konjugates zugegeben.
b) Daraufhin wird ein homologes Polyepitop des Liganden für Rezeptor A (oder Ligand) zugegeben.
c) Zu dem Gemisch von b) wird eine bekannte Menge an Rezeptor B (Anti-Marker) und eine bekannte Menge an Signalmolekül zugegeben, wodurch freie Bindungsstellen am Rezeptor B abgesättigt werden, und die Menge der nicht-gebundenen Signalmoleküle durch geeignete analytische Methoden bestimmt.

Das obige Verfahren kann in der angegebenen Reihenfolge, jedoch auch in andersartiger Anordnung, wie sie dem Fachmann geläufig ist, durchgeführt werden.

Einer der Hauptvorteile dieses Verfahrens liegt darin, dass eine Trennung gebundener und freier Anteile an Signalmolekül nicht erforderlich ist. Dies schliesst jedoch die Anwendung einer solchen Trennstufe bei einem Test nach der Inkubation nicht aus. Eine Trennung der Anteile oder Fraktionen kann zuweilen wünschenswert sein, um Substanzen in der Probe zu entfernen, die den Nachweis des freien Signalmoleküls stören können. Die Trennung kann unter Anwendung irgendeiner der vielen für den Radioimmunotest beschriebenen Methoden, einschliesslich Gelfiltration, Adsorption und Ionenaustauschchromatografie, fraktionierte Fällung, Solid-Phase-Trennung und Elektrophorese durchgeführt werden.

I. Synthese der Reagentien

A) Homologe Polyepitope

Synthese von Testosteron-IgG-Konjugat

5,0 mMol Testoteron-3-carboxy-methyloxim werden in 10 ml trockenem Dimethylsulfoxid gelöst. Zu dieser Lösung gibt man 10 ml trockenes Tetrahydrofuran. Die Lösung wird auf 0°C gekühlt. Im folgenden fügt man 6,0 mMol Tri-n-butylamin und daraufhin 5,0 mMol Chlorameisensäure-isobutylester zu. Nach 15 Minuten erfolgt Zugabe von 0,1 mMol IgG. Nach 2-stündigem Stehenlassen bei Raumtemperatur ist die Reaktion beendet. Zu dem Reaktionsgemisch gibt man in der Kälte Wasser zu. Der Niederschlag wird abgetrennt, das Filtrat dialysiert und daraufhin durch Sephadex-Säulenchromatografie gereinigt.

Synthese von Testosteron-$\varepsilon$-aminocapronsäure-IgG-Konjugat:

5,0 mMol Testosteron-3-carboxy-methyloxim werden in 10 ml trockenem Dimethylsulfoxid aufgelöst. Zu dieser Lösung gibt man 10 ml trockenes Tetrahydrofuran. Die Lösung wird auf 0°C gekühlt. Im folgenden fügt man 6,0 mMol Tri-n-butylamin und daraufhin 5,0 mMol Chlorameisensäure-isobutylester zu. Nach 15 Minuten erfolgt Zugabe von 5,0 mMol $\varepsilon$-Aminocapronsäure. Unter Rühren wird die Reaktion 2 h lang bei Raumtemperatur fortgesetzt. Das Reaktionsgemisch wird daraufhin wieder auf 0°C gekühlt und 5,0 mMol Tri-n-butylamin und im folgenden 5,0 mMol Chlorameisensäure-isobutylester zugegeben. Nach 15 Minuten erfolgt Zugabe von 0,1 mMol IgG in 20 ml Wasser. Nach 2-stündigem Stehenlassen dialysiert man 16 h gegen Wasser. Nach der chromatografischen Reinigung wird das Dialysat gefriergetrocknet.

Nach dem gleichen Verfahren wurden die folgenden Polyisotope hergestellt:
Testosteron-Albumin-Konjugat,
Testosteron-Polylysin-Konjugat.

**B)** Synthese von Marker-Rezeptor-Konjugaten

Auf die gleiche Weise, wie dies vorstehend für die homologen Polyepitope beschrieben wurde, erfolgt unter Berücksichtigung der entsprechenden stöchiometrischen Verhältnisse die Herstellung der folgenden Marker-Rezeptor-Konjugate:

Methotrexat/Anti-Testosteron-Antikörper (IgG-Fraktion des entsprechenden Antiserums),
Methotrexat/Anti-IgG-Antikörper,
Pepstatin/Anti-IgG-Antikörper,
Pepstatin/Anti-Testosteron-Antikörper,
Fluorescein/Anti-IgG-Antikörper,
NADPH/Anti-IgG-Antikörper.

**C)** Synthese eines Ligand-Marker-Konjugates:

Im folgenden wird die Synthese eines Ligand-Marker Konjugats beschrieben.

Synthese von Testosteron-Methotrexat-Konjugat
(Methode nach Erlanger)

0,1 mMol MTX (Methotrexat) wurden in 1500 µl DMF und 40 µl tri-n-Butylamin gegeben. Bei ca. 10°C wurden 0,1 mMol Chlorameisensäureester hinzugegeben. Das Reaktionsgemisch wurde 20 Minuten bei 4°C stehen gelassen.

Daraufhin wurden 30 mg AAO (3-Amino-5 α -dihydroandrostan-17ß-ol) zugegeben. Nachdem weitgehende Auflösung erfolgte, wurden 210 µl 1N NaOH zugesetzt. Das Reaktionsgemisch wurde 1 Stunde bei Raumtemperatur stehen gelassen. Daraufhin wurden 100 µl 1N NaOH zugegeben und wieder 1 Stunde bei Raumtemperatur stehen gelassen. Das Gemisch wurde dann durch Glasfasern in der Pipette filtriert; zum Filtrat wurden 3 ml Wasser zugegeben. Es bildete sich ein Niederschlag. Dieser wurde am nächsten Tag abzentrifugiert und 20 ml Ethanol zugegeben, worauf sich der Niederschlag in der Wärme auflöste. Diese Lösung wurde dann portionsweise eingefroren.

II.    Bildung von Antikörpern gegen Methotrexat

Zur Bildung von Antikörpern wurden Kaninchen mit Methotrexat/Albumin-Konjugat in Adjuvans subkutan immunisiert.

Nach dreimaliger Boosterung und einer Inkubationszeit von 6 Wochen wurde aus dem Serum der Tiere der gegen Methotrexat gebildete Antikörper gewonnen.

III. A) <u>Bestimmung von Testosteron mit Hilfe des erfindungsgemässen Verfahrens</u>

Die Testosteronlösung, welche Methotrexat-Antikörper-Konjugat (Rezeptor A gegen Testosteron)
enthielt, wurde jeweils 30 Minuten lang mit
Testosteron-IgG-Konjugat und unterschiedlichen
Mengen an Testosteron inkubiert.

Daraufhin wurde der gegen Methotrexat gebildete Antikörper (Rezeptor B) im Überschuss zugegeben und wiederum
10 Minuten lang inkubiert. Dann erfolgte Zugabe von
Methotrexat, worauf die Probenlösung 10 Minuten lang
stehen gelassen wurde.

Die Bestimmung von nicht-gebundenem Methotrexat erfolgte im vorliegenden Fall durch Zugabe von DHF-
Reduktase und NADPH (im Überschuss). Es bildet sich
dabei ein stabiler ternärer Komplex (D. A. Matthews
et al in Journal of Biological Chemistry, Band 253,
1978, Seiten 6946 bis 6954). Die Bestimmung von freiem
NADPH erfolgte mittels Extinktionsmessungen.

Bei der Bestimmung von sehr geringen Konzentrationen
an Testosteron ($<$ p-Mol-Bereich) wurde die Bestimmung
von NADPH mit Hilfe der Recycling-Methode durchgeführt.

Unter diesen Bedingungen wurde in Abhängigkeit von der
eingesetzten Testosteronmenge eine Änderung der Extinktion von NADPH bzw. eine Änderung der Reaktionsgeschwindigkeit der Recycling-Reaktion gemessen.

III. B) Bestimmung von IgG mit Hilfe des erfindungsgemässen Verfahrens

Die Testlösung, welche Fluorescein-Antikörper-
Konjugat (Rezeptor A gegen IgG) enthielt, wurde
mit unterschiedlichen Mengen an IgG jeweils
60 Minuten lang inkubiert.

Dazu wurde im Überschuss der gegen Fluorescein
gebildete Antikörper (Rezeptor B) zugegeben und
10 Minuten lang inkubiert.

Unter diesen Bedingungen wurde in Abhängigkeit
von der eingesetzten IgG-Menge eine Änderung
der Fluoreszenz von Fluorescein im Konjugat
gemessen.

III. C) Bestimmung von Testosteronbindungs-Globulin
(TeBG)

Die Testprobe, welche Methotrexat-Anitkörper-Konjugat (Rezeptor A gegen Testosteron) enthielt,
wurde mit Testosteron-IgG-Konjugat und unterschiedlichen Mengen an TeBG 30 Minuten lang inkubiert. Dazu wurde im Überschuss der gegen
Methotrexat gebildete Antikörper (Rezeptor B)
zugegeben und wiederum 10 Minuten lang inkubiert.

Darauf folgte Zugabe von Methotrexat, worauf die
Probelösung 10 Minuten lang stehen gelassen wurde.

Die Bestimmung von nicht-gebundenem Methotrexat
wird im vorstehenden Fall durch Zugabe von DHF-
Reduktase und NADPH (im Überschuss) durchgeführt.

Es bildet sich dabei ein stabiler ternärer Komplex aus. Die Bestimmung von freiem NADPH erfolgt mittels Extinktionsmessungen.

In Abhängigkeit von der zugegebenen Menge an TeBG wurde die Extinktionsänderung von NADPH bestimmt.

Patentansprüche

1. Verfahren zur Bestimmung von Liganden oder Rezeptoren mittels Kompetitionsreaktion unter Verwendung von spezifisch bindendem Rezeptor A und einem Konjugat, dadurch gekennzeichnet, dass
   a) als Konjugat ein Marker-Rezeptor A-Konjugat und
   b) zur Bindung der Markerkomponente von nicht reagiertem Marker-Rezeptor A-Konjugat, ein spezifisch bindender Rezeptor B zugegeben wird,
   c) gegebenenfalls freie Bindungsstellen am Rezeptor B durch Zugabe von Markermolekülen abgesättigt werden, und
   d) die Menge von nicht-gebundenem Marker als Signalmolekül analytisch bestimmt wird.

2. Verfahren gemäss Anspruch 1, dadurch gekennzeichnet, dass es auf homogene oder heterogene Weise durchgeführt wird.

3. Verfahren nach Anspruch 1 oder 2, dadurch g e - k e n n z e i c h n e t , dass man als Ligand ein Antigen,Hapten, Hormon, einen pharmakologischen Wirkstoff oder einen Rezeptor einsetzt.

4. Verfahren nach Ansprüchen 1 bis 3, dadurch g e - k e n n z e i c h n e t , dass man als Rezeptor A : einen Antikörper und/oder ein Fab-Fragment verwendet.

5. Verfahren nach Ansprüchen 1 bis 4, dadurch g e - k e n n z e i c h n e t , dass als Markerkomponente in dem Marker-Rezeptor A-Konjugat bzw. als Signalmolekül ein Enzym , ein Enzymsubstrat, ein Enzymmodulator, wie -aktivator oder -inhibitor, ein Coenzym, ein Lumineszenz- oder Fluoreszenzmolekül verwendet wird.

6. Verfahren nach Anspruch 5, dadurch g e k e n n - z e i c h n e t , dass als Markerkomponente bzw. als Signalmolekül Methotrexat, NADPH oder ATP dient.

7. Verfahren zur Bestimmung von Liganden oder Rezeptoren mittels Kompetitionsreaktion unter Verwendung von spezifisch bindendem Rezeptor A und einem Konjugat, dadurch g e k e n n z e i c h n e t , dass das Konjugat aus Ligand und Markerkomponente gebildet ist.

8. Verfahren nach Ansprüchen 1 bis 7, dadurch g e - k e n n z e i c h n e t , dass als Rezeptor B ein den Marker bzw. die Markerkomponente im Marker-Rezeptor A-Konjugat spezifisch bindender Rezeptor verwendet wird.

9. Verfahren gemäss einem oder mehreren der vorangehenden Ansprüche, dadurch g e k e n n z e i c h n e t , dass zur Bestimmung von Liganden mit einem Molekulargewicht < 100.000 ein homologes Polyepitop mit dem Liganden gebildet wird.

10. Verfahren gemäss Anspruch 9, dadurch g e k e n n - z e i c h n e t , dass das Polyepitop unter Verwendung von künstlichen Polymeren, Polyaminosäuren, Polysacchariden oder Proteinen gebildet wird.

11. Verfahren gemäss Ansprüchen 1 bis 10, dadurch g e - k e n n z e i c h n e t , dass zur besseren sterischen Hinderung Rezeptoren, insbesondere Antikörper von verschiedenen Tierspezies gegen Rezeptor A und/ oder Rezeptor B verwendet werden.

12. Verfahren gemäss Ansprüchen 1 bis 11, dadurch g e - k e n n z e i c h n e t , dass zur Bindung des Liganden ein Gemisch von Marker-Rezeptor A-Konjugat und Rezeptor A zugegeben wird.

13. Verwendung von spezifisch bindenden Rezeptoren A und B, wobei der Rezeptor A gegen die Ligandkomponente, der Rezeptor B gegen die Markerkomponente im Marker-Rezeptor A-Konjugat gerichtet ist, zur Bestimmung von Liganden.

14. Test-Kit zur Durchführung des Verfahrens gemäss Ansprüchen 1 bis 13, dadurch g e k e n n z e i c h n e t , dass dieser im Gemisch oder voneinander getrennt die Rezeptoren A und B, ein Marker-Rezeptor A-Konjugat, Markersubstanz sowie die Substanzen des Nachweissystems zur Bestimmung des Markers enthält.